# EUROPEAN PATENT APPLICATION

(11) **EP 4 825 943 A1**
(43) Date of publication of application: **21.10.2026**
(21) Application number: 25170641.2
(22) Date of filing: 15.04.2025
(51) Int. Cl.: C09K 11/06, C07F 15/00, C09K 11/07, G01N 33/533, G01N 33/58

(54) **NEW COMPOUNDS FOR SIGNAL AMPLIFICATION IN ELECTROCHEMILUMINESCENCE (ECL) SYSTEMS**

(71) Applicant: UNIVERSITA' DEGLI STUDI DI MILANO, 20122 Milano (IT); Alma Mater Studiorum Universita di Bologna, 40126 Bologna (IT)
(72) Inventor: DE COLA, Luisa, 20122 MILANO (IT); RIMOLDI, Isabella Silvia, 20122 MILANO (IT); FACCHETTI, Giorgio, 10122 MILANO (IT); ARNAL VALLÉS, Lorenzo, 10122 MILANO (IT); MENDUTI, Luigi, 10122 MILANO (IT); POTENTI, Simone, 10122 MILANO (IT); VALENTI, Giovanni, 40126 BOLOGNA (IT); PRODI, Luca, 40126 BOLOGNA (IT); DI GIOSIA, Matteo, 40126 BOLOGNA (IT); CALVARESI, Matteo, 40126 BOLOGNA (IT); RAMPAZZO, Enrico, 40126 BOLOGNA (IT)
(74) Representative: Robba, Pierpaolo

(57) **Abstract**

The present invention relates to new compounds that can be used as luminophores in ECL-based detection systems for obtaining an improved signal amplification in ECL-based detection system. These new compounds include a luminophore unit in the form of a metal complex and a chain including a cleavable bond. Thanks to the breaking of the cleavable bonds, the luminophore units are no longer constrained close to one another, preventing any quenching effect, and the freely diffusing luminophore units activate the ECL catalytic pathway that results very efficient for the analytical signal generation. The present invention also relates to the use of the above-mentioned compounds for performing an ECL reaction, namely for the detection of pathogens, bacteria, viruses, parasites, RNA, DNA, biomolecules and other small analytes through ECL-based detection systems.

## Description

### Technical field of the invention

The present invention relates to new compounds that can be used as luminophores in ECL-based detection systems.

More in detail, the present invention relates to new compounds allowing obtaining an improved signal amplification in ECL-based detection system.

The present invention also relates to the use of the above-mentioned compounds for the detection of pathogens, bacteria, viruses, parasites, RNA, DNA, biomolecules, biomarkers and small analytes through ECL-based detection systems.

### Prior art

Electrochemiluminescence or electrogenerated chemiluminescence (ECL) is a kind of luminescence produced during electrochemical reactions in solutions.

In ECL process, electrochemically generated species undergo high-energy electron transfer reaction to form species in an excited state that then emit light upon relaxation to a lower-level state. The wavelength of the emitted photon of light corresponds to the energy gap between these two states.

Since in the ECL mechanism the light emission is triggered by an electrochemical reaction, it does not suffer from the background signal originated by the scattered light or sample autofluorescence that is present in photoluminescence-based - and in particular in fluorescence-based - optical assays.

The absence of light excitation also simplifies the design and fabrication of portable, low-cost devices.

In addition, the reaction can be easily controlled by adjusting the potential applied at the electrode and enhanced selectivity of ECL analysis can be reached by varying the electrode potential.

As a result, ECL has proved to be a powerful analytical technique and is widely used in several technical fields, including immunoassay, food and water testing, biowarfare agent detection and so on.

In case of detection of an analyte from a biological sample, ECL is usually carried out in an aqueous medium and involves the provision of a luminescent species (luminophore) and a co-reactant.

Typical ECL immunoassays exploit ruthenium-based complexes or iridium-based complexes as luminophores and tri-n-propylamine (TPrA) as co-reactant.

A widely used ruthenium-based complex is tris(2,2'-bipyridine)ruthenium(II) ([Ru(bpy)₃]²⁺).

ECL of [Ru(bpy)₃]²⁺ in pure water with TPrA as a co-reactant was developed in 1990 and used in the first ECL clinical analyzer, commercialized by IGEN (Rockville, MD) in 1994. [Ru(bpy)₃]²⁺ electrochemical and photophysical properties along with its low toxicity, and good solubility in a wide range of solvents, including aqueous buffer solutions, make it particularly attractive for ECL-based biological assays.

Over the past 30 years, ruthenium polypyridyl complexes have emerged as the predominant luminophores, having been the focus of extensive research. Their highly favorable electrochemical properties - both in terms of stability and oxidation potentials - can produce an intense emission that is well-suited for practical ECL applications.

However, ruthenium polypyridyl complexes are not free from limitations (for instance in terms of low intrinsic photoluminescence quantum yields), so that considerable efforts have been made to find alternatives.

Therefore, both alternative ruthenium-based complexes and iridium-based complexes have been intensively investigated.

Examples of suitable ruthenium-based and/or iridium-based complexes can be found, for instance, in documents WO 2011/067401, WO 2012/107419 and WO2014/019708.

In order to enhance the signal-to-noise ratio of ECL-based biosensors, it is known to replace a single [Ru(bpy)₃]²⁺ unit or similar unit based on another suitable metal complex with multinuclear complexes or polymeric systems embedding multiple identical emitting units. However, the enhancement in ECL sensitivity arising from such approach is not directly proportional to the increase in the number of emitting units used.

This is allegedly due to the quenching effect exerted on the luminophores in the excited state by the luminophores at ground state (or in the oxidized or reduced form) because of their proximity, which also limits the possibility of further increasing the number of emitting units employed.

In view of the above, an object of the present invention is to provide new complexes allowing achieving better signal amplification and improved sensitivity in ECL systems. Another object of the present invention is to provide new complexes particularly suitable for the detection of pathogens, bacteria, viruses, parasites, RNA, DNA, biomolecules and small analytes through ECL.

These and other objects are achieved by the complexes as claimed in the appended claims.

### Summary of the invention

The present invention relates to a compound having a general formula (I)

A-B-C (I)

wherein
A is a luminophore unit in the form of a metal complex,
B is a linker including a cleavable bond,
C is a conjugating group or an antibody for the sandwich assay.

Indeed, the research for improved ECL performance has always been focused on systems including chemically inert labels.

Instead, the Applicants have surprisingly found out that a higher sensitivity in ECL systems, namely in ECL systems applied to analyte detection, can be achieved by using cleavable emitting probes.

By adopting cleavable emitting probes, two main advantages can be achieved:
- the luminophore units are no longer constrained close to one another, preventing any quenching effect;
- the freely diffusing luminophore units activate the ECL catalytic pathway (homogeneous mechanism) that results very efficient for the analytical signal generation.

As a result, a signal amplification that is proportional to the increase in the number of emitting units used can be aimed.

With respect to the increase in the number of emitting units, it should be noted that the invention (due to the release of luminophores upon breaking of the cleavable bonds) allows effectively using multinuclear complexes, without the disadvantages of prior solutions related to the quenching effect.

The emitting units can be anchored on a platform so as to have hundreds of complexes on a small substrate.

For instance, hundreds of complexes can be used for decorating the surface of a nanoparticle, such as a silica nanoparticle.

The invention relies upon the concept of breaking the linkers that connect all the metal complexes, upon application of a selected stimulus, making the luminophores free in solution that can act as monomers and do not interfere with one another (thus avoiding any quenching effect).

Theoretical models and experimental tests have shown that by adopting the compound as luminophore, the behavior of the ECL system shifts from a heterogenous mechanism to a more effective homogeneous mechanism as the cleavable bonds are broken and the metal complexes are released.

Advantageously, experimental tests proved that the cleavable bonds are rapidly broken (within few hundreds of milliseconds after the application of the applied stimulus), so that the homogeneous mechanism readily appears.

Since the conventional ECL systems based on the heterogeneous mechanism usually rely on high concentrations of co-reactant, while homogeneous ECL systems generally show a better performance with more diluted co-reactant solutions, the invention allows implementing ECL systems with a lower co-reactant concentration.

The invention may be implemented by using different complexes, differing in the numbers, type of ligands and metal units, as the triggering of the amplification only depends on the nature of the stimulus (and in particular on the electrochemistry process) and is independent of any other variable related to metal complex design.

Nevertheless, in preferred embodiments of the invention, A is a mono-nuclear metal complex having a general formula (II) as shown below 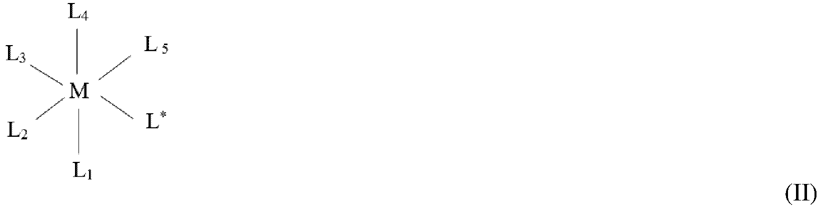 wherein
M is a metal ion
L₁ - L₅ are functional ligands bound to the central metal ion to form the metal complex L* is an ancillary ligand to which linker B is connected.

Nevertheless, in alternative embodiments of the invention, the metal complex may be a poly-nuclear metal complex, in particular a di-nuclear metal complex.

In embodiments of the invention, the metal complex comprises one or more metals selected from ruthenium (Ru(II)), rhodium (Rh(I)), osmium (Os(II)), zinc(Zn(II)), chromium (Cr(III)), platinum (Pt), palladium (Pd), iridium (Ir(III)), copper (Cu(I)), rhenium (Re(I)), aluminum (Al(III)), gallium (Ga(III)) and the rare earth metals.

In a particularly preferred embodiment of the invention, metal is selected among ruthenium and iridium. Therefore, in a particularly preferred embodiment of the invention, A is selected among ruthenium-based complexes and iridium-based complexes.

The ligands - namely, the ancillary ligand - may be monodentate, bi-dentate or generally polydentate linkers. Macrocyclic, monocyclic or, optionally bridged, polycyclic moieties may also be used.

Preferred ligands are selected from substituted or unsubstituted: 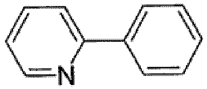 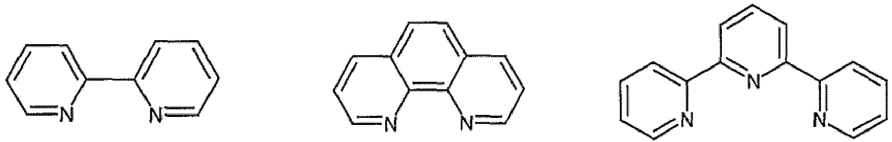 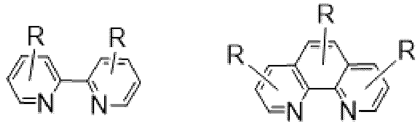 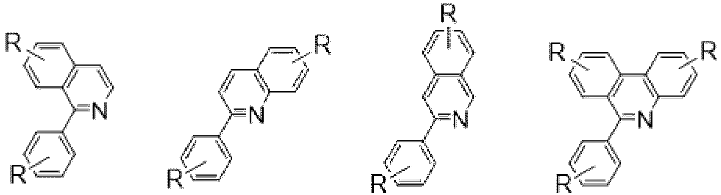 wherein R is selected from -CH₂OH, -CH₂O-(CH₂)NSO₃⁻, -CH₃, -H, -CH₂O-Si(O-(CH₂)NCH₃)₃, -(CH₂)n, -F, -(CF₂)NCF₃, -OMe, -CF₃, -NO₂, -NH₂, -SH, -CHO, -COOH and -CN.
or are defined as a moiety of the general formula (III): 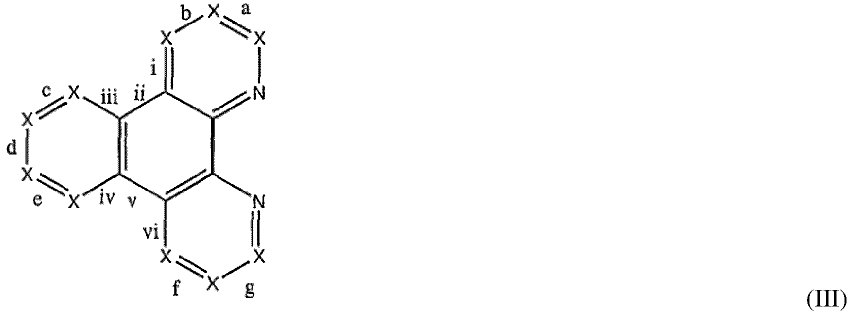 wherein X is independently CH or N, preferably at least one of the groups X being N, and the bonds a, b, c, d, e, f, and g, and the combination of bonds i/ii/iii and iv/v/vi are optionally condensed with a benzene group or a condensed aromatic moiety, wherein aromatic carbon atoms may be replaced by nitrogen atoms and wherein the complexing moiety may be substituted with C₁₋₆ alkyl, C₂₋₆ alkenyl, C₂₋₆ alkynyl, C₃₋₄ alkylene, CN, halogen, COOH, C₁₋₃ alkyl-COOH, NO₂, NH₂, or a pending group for further functionalization or complexation.

More preferred are the complexes wherein two of the groups X are N.

Macrocyclic moieties such as phthalocyanine and porphyrin moieties are also preferred. Moreover, the invention may be implemented by using different linkers and different cleavable bonds, as far as the linker can be broken upon application of a selected stimulus (such as light, pH, voltage, and so on).

In preferred embodiments of the invention, B is an organic chain such as an aromatic chain or an aliphatic chain.

In the compound according to embodiments of the present invention, the linker B, that includes the cleavable bond, comprises a backbone length of between 1 and 200 atoms.

In one embodiment, the B has as a backbone a straight or branched saturated, unsaturated, unsubstituted or substituted C1-C200 alkyl chain, or a 1 to 200 atom chain consisting of carbon atoms, substituted carbon atoms and/or one or more atoms selected from O, N, P and S, or substituted N, P, S atoms, or a chain as described before with the backbone containing one or more cyclic or heterocyclic aromatic or non-aromatic ring systems.

In one embodiment, B has as a backbone a straight or branched saturated, unsaturated, unsubstituted or substituted C1-C100 alkyl chain, or a 1 to 100 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one embodiment, B has as a backbone a straight or branched saturated, unsaturated, unsubstituted or substituted C1-C50 alkyl chain, or a 1 to 50 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one further embodiment, B has as a backbone a straight or branched saturated, unsaturated, unsubstituted or substituted C1-C20 alkyl chain, or a 1 to 20 atom chain consisting of carbon atoms, substituted carbon atoms and/or one or more atoms selected from O, N, P and S, or substituted N, P, or S atoms, or a chain as described before with the backbone containing one or more cyclic or heterocyclic aromatic or non-aromatic ring systems.

In one embodiment, B has as a backbone a straight or branched saturated, unsaturated, unsubstituted, substituted C1-C20 alkyl chain, or a C1-C20 arylalkyl chain (wherein e.g. a phenylene ring accounts for a length of four carbon atoms), or a 1 to 20 atom chain with a backbone consisting of carbon atoms, substituted carbon atoms and/or one or more atoms selected from O, N, P and S, or substituted N, P, or S atoms, or a 1 to 20 atom chain, or with a backbone consisting of carbon atoms, substituted carbon atoms and one or more atoms selected from O, N, P and S, or substituted N, P, or S atoms comprising at least one aryl, heteroaryl, substituted aryl or substituted heteroaryl group (wherein e.g. a phenylene ring accounts for a length of four atoms).

In one embodiment, B has as a backbone a saturated C1 -C12 alkyl chain, or a C1-C12 arylalkyl chain, or a 1 to 12 atom chain with a backbone consisting of carbon atoms, substituted carbon atoms and one or more atoms selected from O, N, P and S, or substituted N, P, or S atoms, or a 1 to 12 atom chain with a backbone consisting of carbon atoms, substituted carbon atoms and one or more atoms selected from O, N, P and S, or substituted N, P, or S atoms comprising at least one aryl, heteroaryl, substituted aryl or substituted heteroaryl group (wherein e.g. a phenylene ring accounts for a length of four atoms).

In one embodiment, the linker B, that includes the cleavable bond, comprises one or more amino acid(s).

In one embodiment, the linker B, that includes the cleavable bond, comprises one or more nucleotide(s).

In one embodiment, the linker B, that includes the cleavable bond, comprises a peptide chain.

In a preferred embodiment of the invention, B includes a cleavable bond that can be broken upon application of voltage, preferably a low voltage.

In a particularly preferred embodiment of the invention B includes a redox-sensitive bond, i.e., a bond that is broken as the result either of oxidation or of reduction.

Examples of bonds that can be broken by oxidation include aliphatic peroxide or tertiary amine.

In a particularly preferred embodiment of the invention, B includes a bond that can be broken by reduction, and most preferably a disulfide bond, as the S-S moiety is easily reduced (for an applied voltage lower than - 1,5 V) to thiols.

Nevertheless, in alternative embodiments of the invention, cleavable bonds that can be broken upon application of a different chemical stimulus or upon application of a physical stimulus (e.g., temperature) can be envisaged.

In a particularly preferred embodiment of the invention, the compounds of general formula (I) are selected among the compounds according to formulas IV to VI shown below

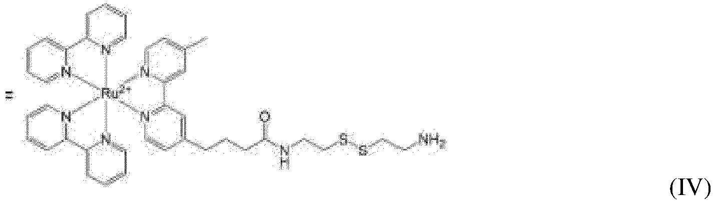

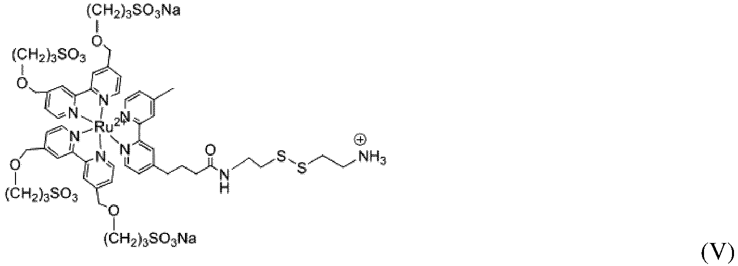

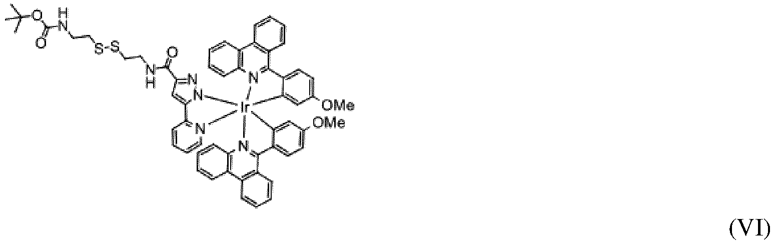

In preferred embodiments of the invention, C may be, for instance, amine (-NH₂), amide, azide (-N3), carboxylate (-COOH), hydroxy (-OH), thiol (-SH), alkyne (-C≡CH), alkene (-CH=CH2) , carbamate (-NH-CO-OR/RNH-CO-O-) , isocyanate (-NCR), tri-alkoxysilane group (-Si(OR)3) carboxyl group, streptavidin or N-hydroxysuccinimide group, aldehyde, carboxylic acid, carboxylic acid ester, epoxide, N- hydroxysuccinimide ester, halogen, hydrazine, hydroxyl, sulfhydryl, maleimido, alkynyl, isothiocyanate or phosphoramidite. In other preferred embodiments of the invention, C could link the luminophore to an antibody feasible for the sandwich immunoassay system or DNA/RNA sequence.

As mentioned above, the compound according to invention is used in combination with a co-reactant in an ECL reaction.

In embodiments of the invention, the co-reactant is selected among primary or secondary or tertiary amines, oxalate and hydrogen peroxide.

In a preferred embodiment of the invention, tri-n-propylamine (TPrA) is used as a co-reactant.

In case TPrA is used as co-reactant, experimental tests proved that its concentration can be decreased from 180 mM (as found in current commercial solutions) down to 40 - 50 mM. This represents a remarkable advantage in the case of TPrA and other toxic co-reactants. Thanks to the possibility of strikingly increasing the sensitivity of the ECL system, the invention can advantageously be employed in detecting pathogens, bacteria, viruses, parasites, RNA, DNA, biomolecules, biomarkers and other small analytes in biological samples.

It will be evident to the person skilled in the art that the embodiments described in detail in the present disclosure should in no way be understood in a limiting sense, and that numerous modifications and variants are possible without thereby departing from the scope of protection as defined by the appended claims.

### Brief description of the drawings

Theoretical models and experimental tests relating to the complexes according to embodiments of the invention are shown in the annexed drawings, in which:
- Figure 1a shows a bead carrying a luminophore according to prior art as well as a schematic representation of the corresponding ECL mechanism;
- Figure 1b shows a bead carrying a luminophore according to an embodiment of the invention as well as a schematic representation of the corresponding ECL mechanism;
- Figure 2a is an ECL image obtained using a luminophore according to prior art;
- Figure 2b is an ECL image obtained using a luminophore according to an embodiment of the invention;
- Figure 3 shows the ECL intensity profiles obtained using a luminophore according to prior art and using a luminophore according to an embodiment of the invention, respectively;
- Figure 4a is a further ECL image obtained using a luminophore according to prior art;
- Figure 4b is a further ECL image obtained using a luminophore according to an embodiment of the invention;
- Figure 5 shows the ECL signal decay profiles obtained using a luminophore according to prior art and using a luminophore according to an embodiment of the invention, respectively;

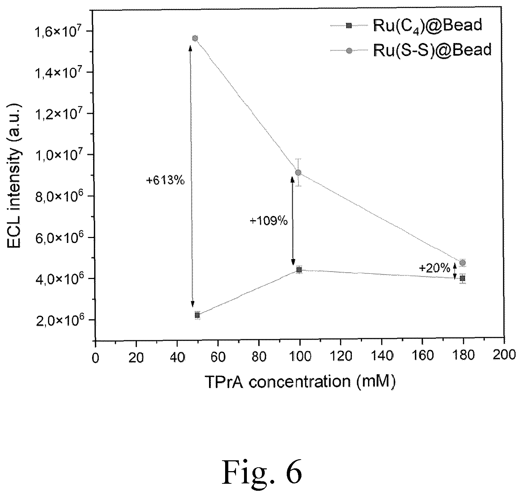

- Figure 6 shows the ECL intensity for different co-reactant concentrations obtained using a luminophore according to prior art and using a luminophore according to an embodiment of the invention, respectively;
- Figure 7 shows the pathway for synthesis of compound having formula (V);
- Figure 8 shows the pathway for synthesis of compound having formula (VI).

### Experimental tests and results

### Feasibility test

In order to verify the feasibility of the proposed reaction mechanism, a series of [Ru(bpy)₂(bpy-cys-NH₂)]²⁺, a [Ru(bpy)3]²⁺ derivative incorporating a disulfide moiety anchored on the ancillary bipyridine, were synthesized and a bead including a complex according to the invention (Ru(S-S)@Bead) was obtained by decorating a second 2.8 µm magnetic nanoparticle with carboxylic groups by exploiting the terminal amine functionalities of [Ru(bpy)₂(bpy-cys-NH₂)]²⁺.

A 'release experiment' was carried out by the Applicants, combining electrochemistry with inductively coupled plasma mass spectrometry (ICP-MS).

This experiment was carried out on a 0.3 M Ru(S-S)@Bead deposited on a glassy carbon (GC) electrode housed in a three-electrode electrochemical cell filled with 1 mL of phosphate buffer (PB) solution with 180 mM TPrA (pH 6.8).

A single measurement cycle involves a two-step chronoamperometry consisting in an initial step at 0 V followed by a second step at 1.4 V. This potential ensures diffusion-controlled oxidation of TPrA.

Following this step, the co-reactant solution is recovered, the electrode surface is meticulously cleaned, and the cycle is completed.

The experiment consists of ten such cycles. Each cycle utilizes fresh, functionalized beads on the electrode surface, while the same co-reactant solution is employed throughout the whole experiment.

This approach concentrates the luminophore in solution, facilitating its detection by ICP-MS.

The ruthenium concentration in the release solution is determined to be 1.09 + 0.07 g/L, supporting the feasibility of the proposed reaction mechanism in co-reactant ECL working conditions.

### Comparative study 1

In order to evaluate the surprising result that can be obtained by a compound according to the invention, a series of [Ru(bpy)₂(bpy-cys-NH₂)]²⁺, a [Ru(bpy)3]²⁺ derivative incorporating a disulfide moiety anchored on the ancillary bipyridine, were synthesized and ECL immunoassay methodology using magnetic microbeads on an electrode was implemented.

As reference compound, a conventional complex, namely [Ru(bpy)₂(bpy-C₄-NH₂)]²⁺, including no cleavable bond, was used.

Accordingly, a reference bead (Ru(C₄)@Bead) was obtained by decorating a first 2.8 µm magnetic nanoparticle with carboxylic groups by exploiting the terminal amine functionalities of [Ru(bpy)₂(bpy-C₄-NH₂)]²⁺, a bead including a complex according to the invention (Ru(S-S)@Bead) was obtained by decorating a second 2.8 µm magnetic nanoparticle with carboxylic groups by exploiting the terminal amine functionalities of [Ru(bpy)₂(bpy-cys-NH₂)]²⁺, both beads underwent an ECL immunoassay methodology using TPrA as co-reactant and the results were compared.

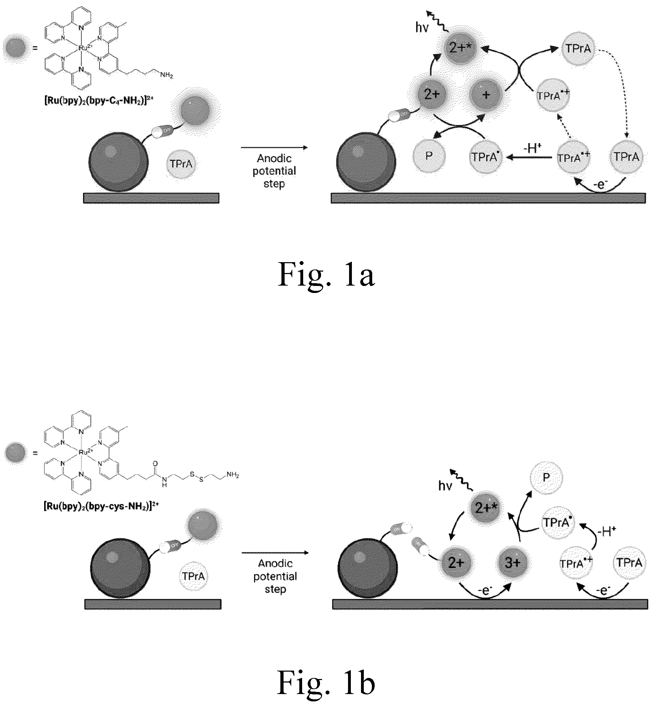

The above-mentioned beads and the related mechanisms of light emission are shown in Figures 1a and 1b, in which the unbreakable behavior of (Ru(C₄)@Bead) and the breakable behavior of (Ru(S-S)@Bead) have been clearly illustrated.

With reference to Figure 1a, the luminophore is anchored to the bead by the terminal amine moiety available for conjugation. Upon application of a suitable anodic potential, the traditional (heterogeneous) mechanism is activated, resulting in the ECL emission of the Ru(II) derivative. Even upon application of the anodic potential the luminophore remains anchored to the bead.

Referring now to Figure 1b, in this case, too, the luminophore is anchored to the bead by the terminal amine moiety available for conjugation until the bead is in a resting state, without any electrochemical stimulus. However, since the luminophore incorporates a disulfide bond, upon application of a suitable anodic potential, the reactivity of the disulfide bond is "switched" on by the co-reactant electrogenerated reducing radicals that cleave the linker due to the reduction of the S-S moiety to thiols, thus releasing the luminophore into the solution. If the applied potential is anodic enough to oxidize also the luminophore, the more effective homogeneous ECL mechanism takes place.

The breakable Ru(S-S)@Bead consistently exhibits a much stronger ECL intensity throughout the measurement compared to the non-breakable Ru(Cs)@Bead, with the enhancement most pronounced as soon as the anodic potential kicks in.

In addition, Ru(S-S)@Bead readily displays intense homogeneous ECL, suggesting that most of the disulfide bonds are cleaved within the first hundreds of milliseconds.

### Comparative study 2

In order to address the ECL behavior of labeled beads, a comparative study between Ru(C₄)@Bead and Ru(S-S)@Bead as defined in Comparative study 1 was carried out by taking advantage of ECL microscopy, enabling direct visualization of the ECL signal. ECL microscopy integrates a potentiostat, which governs the electrochemistry of the investigated system, with an epifluorescence microscope for spatially resolving the ECL signal. This synergy has remodeled ECL into a powerful imaging technique that enables direct observation of objects that spark or influence ECL reactions, providing unparalleled insights to shed light on reaction mechanisms.

In this study, this feature is particularly valuable for imaging the spatial distribution of the ECL signal at single-bead level, obtaining information about the cleavage and diffusion of luminophores within the system.

ECL measurements were conducted by recording the emission of both systems, Ru(C₄)@Bead and Ru(S-S)@Bead, in a 0.3 M PB solution with 180 mM TPrA (pH 6.8). The ECL images of Ru(C₄)@Bead and Ru(S-S)@Bead were captured with an EM-CCD camera by integrating the emission during a two-step chronoamperometry measurement on a GC electrode.

The applied potential sequence consisted of an initial step of 2 s at 0 V vs Ag/AgCl followed by a second step of 43 s at 2.5 V vs Ag/AgCl.

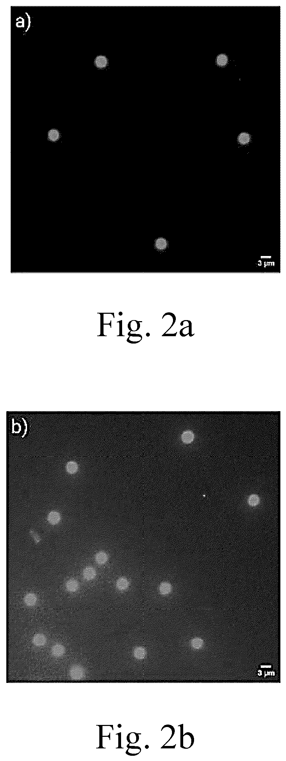

Figures 2a and 2b respectively show the results for Ru(C₄)@Bead and Ru(S-S)@Bead (Magnification X100; objective numerical aperture 1.1; gain 1; sensitivity 250; contrast scale 4000-16000; scale bar 3 µm).

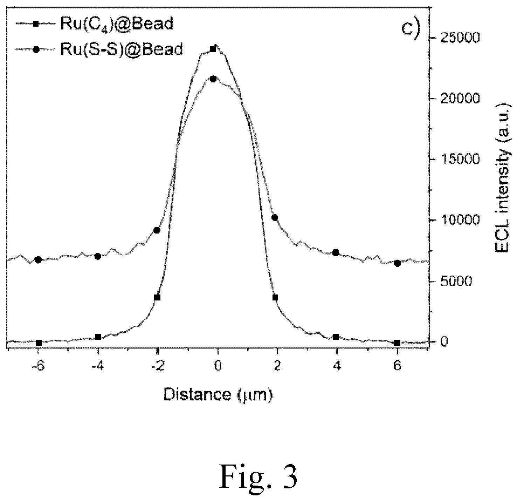

Figure 3 shows the comparison between the single-bead ECL intensity profiles of Ru(C₄)@Bead (grey line) and Ru(S-S)@Bead (red line). Data are averaged over a minimum of five beads.

Ru(C₄)@Bead shows the conventional ECL behavior, with emission strictly confined to the bead surface and negligible background signal (as can be seen in Figure 3).

Any deviation from this typical pattern in Ru(S-S)@Bead can therefore be attributed to the homogeneous ECL following the cleavage of disulfide bridges.

Strikingly, the background emission of Ru(S-S)@Bead shows a clear gradient, intensifying as the beads become closer to one another (as can be seen in Figure 3).

A higher density of beads in a given spatial region appears to translate into a higher concentration of released ruthenium thereby leading to stronger ECL emission.

The release of Ru(II) complexes from the beads is also reflected in the shape of the ECL profiles.

These profiles are computed by extracting ECL intensity values across each pixel along the x-axis (i.e., distance) within a rectangular region of interest (ROI) centered on the bead emission.

A comparison between Ru(C₄)@Bead and Ru(S-S)@Bead profiles shows that Ru(S-S)@Bead shows bright background emission with an intensity around ~7000 a.u., while it is negligible in Ru(C₄)@Bead.

The ECL signal directly generated by beads labeled with the cleavable complex is weaker than that observed for their counterpart. Assuming an equal degree of functionalization in both cases, this behavior can be attributed to the release of luminophores in Ru(S-S)@Bead, that favors the background signal at the expanse of the signal originating from the beads themselves.

In order to follow the ECL temporal evolution so as to monitor the kinetics of disulfide bond cleavage, the ECL decay measurement was also recorded in 0.3 M PB with 180 mM TPrA (pH 6.8) with an EM-CCD camera by capturing a frame every 500 ms for a total duration of 60 s during a two-step chronoamperometry measurement on a GC electrode.

The applied potential sequence consisted of an initial step of 2 s at 0 V vs Ag/AgCl followed by a second step of 58 s at 2.5 V vs Ag/AgCl.

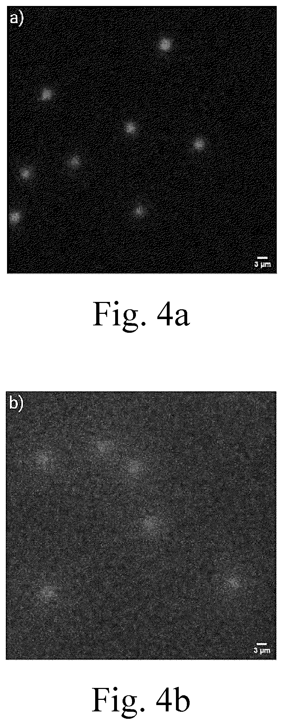

Figures 4a and 4b show the first frame of emission of Ru(C₄)@Bead and Ru(S-S)@Bead, respectively, captured after applying the anodic potential (Magnification X100; objective numerical aperture 1.1; gain 1; sensitivity 250; contrast scale 1550-1900; scale bar 3 µm).

The emission intensity for each frame was retrieved by integrating the signal over a square ROI centered on the bead. The resulting ECL values were then plotted as function of time.

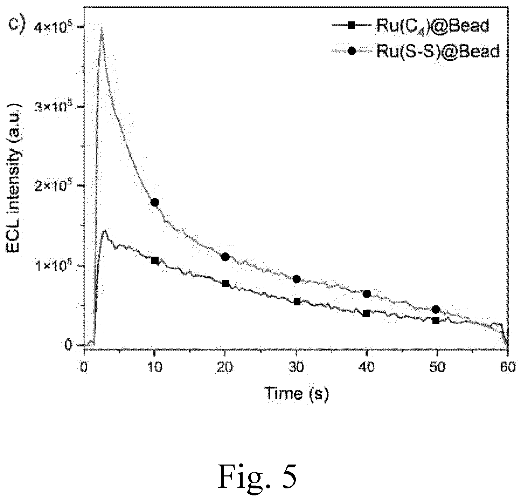

Figure 5 shows the comparison between the single-bead ECL decay of Ru(C₄)@Bead (grey line) and Ru(S-S)@Bead (red line). Data are averaged over a minimum of five beads.

It should be noted that the ROI area includes not only the bead itself but also its immediate chemical environment to account for the contribution of homogeneous ECL beyond the bead edges.

Notably, Ru(S-S)@Bead consistently exhibits a stronger ECL intensity throughout the measurement compared to Ru(C₄)@Bead, with the enhancement most pronounced as soon as the anodic potential kicks in.

This behavior can be explained by close investigation of Figures 4a and 4b. The background emission of Ru(C₄)@Bead is not significative, whereas Ru(S-S)@Bead readily displays intense homogeneous ECL, suggesting that most of the disulfide bonds are cleaved within the first hundreds of milliseconds.

However, the Ru(S-S)@Bead signal experiences a much steeper decay, after which both systems appear to decay at a similar rate (as clearly visible in Figure 5).

This burst stems from the diffusion of cleaved Ru(II) labels. In fact, when the luminophores are still in close proximity to the beads in the first seconds, the complex concentration is high enough for the camera to detect the homogeneous ECL. As these labels diffuse away into the bulk solution, it is likely that their concentration drops below the detection limit.

### Comparative study 3

The impact of different concentrations of co-reactant (TPrA) on the ECL behavior of both Ru(C₄)@Bead and Ru(S-S)@Bead was investigated.

The interest of this comparative study resides in that conventional bead-based systems (heterogeneous) usually rely on high concentrations of TPrA, typically 180 mM, while homogeneous ECL systems generally show a better performance with more diluted co-reactant solutions.

ECL images of both beads on a GC electrode in 0.3 M PB solution with 50 mM, 100 mM or 180 mM TPrA (pH 6.8) were captured and analyzed. The ECL images of Ru(C₄)@Bead and Ru(S-S)@Bead were captured with an EM-CCD camera by integrating the emission during a two-step chronoamperometry measurement on a GC electrode.

The applied potential sequence consisted of an initial step of 2 s at 0 V vs Ag/AgCl followed by a second step of 11 s at 2.5 V vs Ag/AgCl.

With reference to Figure 5, the ECL signal was integrated and the resulting values were plotted as a function of the co-reactant concentration. Data are averaged over a minimum of five beads. The error bars represent the standard error.

As evident from Figure 5, Ru(C₄)@Bead and Ru(S-S)@Bead exhibit two diametrically opposed trends with decreasing TPrA concentration.

While the ECL intensity of the conventional system tends to weaken as the co-reactant concentration drops, the breakable system shows a massive surge in emission under the same conditions. In particular, the ECL signal of Ru(C₄)@Bead remains mostly unchanged from 180 mM to 100 mM TPrA, and eventually plunge at 50 mM.

This behavior is not surprising, as heterogeneous systems require high concentrations of TPrA to overcome the bottleneck caused by their slow oxidation rate. Thus, at 50 mM, Ru(C₄)@Bead might lack enough radicals to express a strong ECL signal: because of the micrometric dimension of the bead, the shuttling of the electrons mediated by the TPrA requires a high concentration of co-reactant. The ECL signal of Ru(S-S)@Bead, instead, steadily grows brighter across the entire concentration range, from 180 mM to 50 mM TPrA. It is noteworthy that labels incorporating a disulfide moiety are cleaved even on a commercially employed electrode for bioanalysis, suggesting that the exploitation of breakable luminophores could be a promising strategy for commercial bead-based biosensors.

Valuable insights to explain the Ru(S-S)@Bead behavior are provided by analysis of the ECL profiles computed from integrated images.

The ratio between the peak of the ECL profiles and the intensity of the corresponding backgrounds progressively diminishes upon decreasing the co-reactant concentration. In other words, the contribution of Ru(II) labels bound on the beads to the overall ECL signal weakens at lower TPrA concentrations, whereas the contribution of the ECL in the background strengthens.

These observations suggest two key points. First, the observed trend is likely to be driven by the homogeneous mechanism. Second, the latter reaction pathway becomes increasingly dominant at lower TPrA concentrations.

It is reasonable to assume, then, that the inverse relationship between the ECL intensity of Ru(S-S)@Bead and the TPrA concentration is defined by the effectiveness of the homogeneous mechanism, which generate the strongest emission in solutions with 40 - 50 mM.

In addition, the signal gain observed in Fig. 5 drops as low as 20% at 180 mM TPrA. Since the majority of the signal gain is attributed to the homogeneous ECL, it is straightforward that its contribution is marginal in the most concentrated co-reactant solution.

As the Ru(S-S)@Bead system adopts both the heterogeneous and the homogeneous mechanisms to generate the ECL signal, the above observations suggest the possibility to tune the reaction mechanism by controlling the co-reactant concentration, forcing the system to walk down a predominantly heterogeneous pathway at concentrated TPrA solutions or to follow the homogeneous mechanism at diluted TPrA solutions.

### Synthesis of exemplary compounds of the invention

### Synthesis of compound including Ru-complex having formula (V)

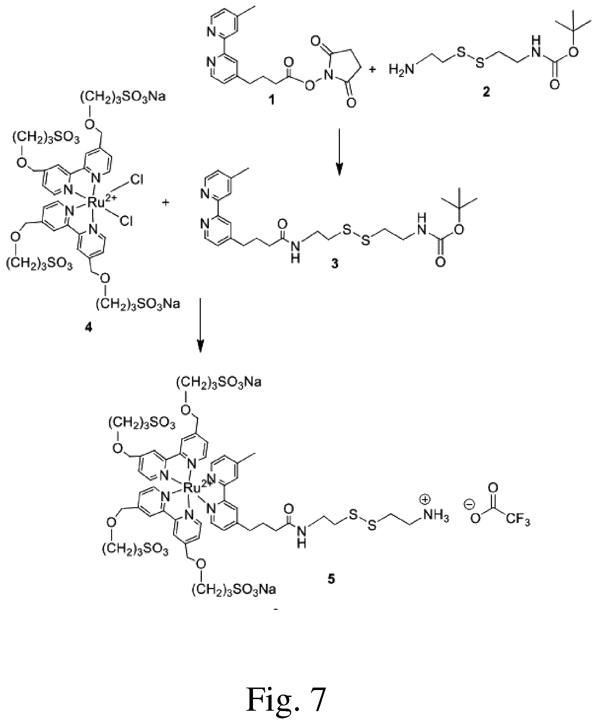

With reference to Figure 7, the reaction pathway for obtaining the compound of formula (III) is shown.

Synthesis of 4-(4'-methyl-[2,2']bipyridinyl-4-yl)butyric acid succinimidyl ester (1) 270 mg of DCC (2.34 mmol) and 140 mg H-hydroxysuccinimide (0.98 mmol) were added to 300 mg (1.17 mmol) of 4-(4'-methyl-[2,2']bipyridinyl-4-yl)-butyric acid in CH₂Cl₂; and stirred at room temperature for 2 hours. After 2 hours, the reaction mixture was filtered and concentrated in vacuo and a clear mixture of powder and oil was obtained. After Combi column chromatography (SiO₂, gradient from 100% Hex to 100% EtOAc) the final product was obtained as a clear oil (270 mg, 0.61 mmol, 76%).

₁H NMR (300 MHz, CD₃0D): δ(ppm) = 8.43 (dd, 2H), 8.12 (d, 2H), 7.03 (d, 2H), 2.70 (m, 6H), 2.54 (t, 2H), 2.30 (s, 3H), 2.04 (t, 2H).

ESI(+)-MS: m/z calculated for C₁₉H₁₉N₃O₄ 353.38; found 354.47 [M + H]⁺.

### Synthesis of tert-butyl (2-((2-aminoethyl)disulfanyl)ethyl)carbamate (2)

Cystamine dihydrochloride (4 g, 17.78 mmol, 1 equiv.) was dissolved in methanol (200 mL) and cooled to 0°C. Triethylamine (7.45 mL, 53.33 mmol, 3 equiv.) was added, and the reaction mixture was stirred for 30 minutes. Boc-anhydride (Boc₂O, 4.05 mL, 17.78 mmol, 1 equiv.) was added dropwise over 10 minutes, and the reaction mixture was allowed to stir for 2 h at 0 °C. The crude mixture was concentrated under reduced pressures, leading to a white precipitate. Then, the white precipitate was filtered and washed with diethyl ether (3x 30 mL). The filter cake was treated with 20 mL of 1 M NaOH solution, and the obtained aqueous phase was extracted with CH₂Cl₂ (2x 25 mL). Both organic layers were combined and washed with water (2x 20 mL) and dried over anhydrous Na₂SO₄ and concentrated in vacuo to yield a yellow oil. (2.4 g, 9.5 mmol, yield 53%).

¹H NMR (300 MHz, CD₃OD): δ (ppm) = 3.39 (t, 2H), 3.01 (t, 2H), 2.83 (dd, 4H), 1.48 (s, 9H).

¹³C NMR (75 MHz, CD₃OD): δ(ppm) = 158.25, 80.11, 41.46, 40.98, 40.71, 39.07, 28.78. ESI(+)-MS: m/z calculated for C₉H₂₀N₂O₂S₂; 252.39; found, 253.13 [M + H]⁺

### Synthesis of tert-butyl(2-((2-(4-(4'-methyl-[2,2'-bipyridin]-4-yl)butanamido)ethyl) disulfanyl) ethyl)carbamate (3)

90 mg (0.25 mmol) of 4-(4'-methyl-[2,2']bipyridinyl-4-yl)butyric acid succinimidyl was dissolved in 3 mL DMF,and added to a solution of 100 mg (excess) of 1Boc-cystamine (0.39 mmol) in 1 mL DMF. After two hours, 0.5 mL of DIPEA was added to ensure deprotonation. The reaction mixture was stirred for 16 hours at room temperature. After 16 hours, the reaction mixture was concentrated in vacuo, taken up in CH₂Cl₂, extracted 2X with a saturated sodium bicarbonate solution, dried over NaSO₄, filtered, and reconcentrated in vacuo.

The crude product was washed with saturated NH₄Cl solution to eliminate the excess of cystamine. The light-yellow oil was obtained as the pure product (35 mg, 0.071 mmol, 32%).

¹H NMR (300 MHz, CD₂Cl₂): δ (ppm) = 8.48 (dd, 2H), 8.26 (d, 2H), 7.15 (d, 2H), 3.52 (dd, J = 6.0 Hz, 2H), 3.40 (dd, J = 6.5 Hz, 2H), 2.88 - 2.68 (m, 6H), 2.44 (s, 3H), 2.23 (t, J = 7.4 Hz, 2H), 2.01 (t, 2H), 1.40 (s, 9H).

¹³C NMR (75 MHz, CD₂Cl₂): δ (ppm) = 172.48, 155.87, 151.66, 149.00, 148.12, 124.62, 123.93, 121.76, 121.11, 79.21, 42.63, 40.64, 38.13, 35.38, 34.63, 28.03, 26.12, 20.96. ESI(+)-MS: m/z calculated for C₂₄H₃₄N₄O₃S₂, 490.21; found 491.21 [M +H]⁺.

### Synthesis of dichlorobis(Sodium 3,3'-(([2,2'-bipyridine]-4,4'-diylbis(methylene)) bis(oxy))bis(propane-1-sulfonate)ruthenium(II) (4)

Under nitrogen atmosphere [2,2'-bipyridine]-4,4'-diyldimethanol (1 g, 4.62 mmol, 1 equiv.) was dissolved in anhydrous DMF (36 mL). At 0°C, NaH (433 mg, 11.55 mmol, 2.5 equiv.) was added, and the reaction mixture was stirred for 0.5 hours at room temperature.

Then, 1,3-propansultone (1.22 mL, 13.9 mmol, 3 equiv.) was added at 0 °C, and the reaction mixture was stirred overnight at room temperature. The reaction was quenched by dropwise addition of H₂O at 0 °C until no further effervescence was observed. At the end of the addition, the crude mixture appeared as a limpid and uncolored solution. The solvent was evaporated under reduced pressure and a white solid was obtained.

The latter was redissolved in hot methanol. The obtained hot mixture was filtered. The filtrate was poured in an Erlenmeyer flask and kept inside a closed container under diethyl ether atmosphere overnight (vapor diffusion precipitation), leading to the spectroscopically pure product as ivory powder (2.32 g, 4.6 mmol, 99% yield).

¹H NMR (300 MHz, D₂0): δ (ppm) = 8.56 (d, J = 5.1 Hz, 2H), 7.91 (s, 2H), 7.47 (d, J = 6.8 Hz, 2H), 4.67 (s, 4H), 3.73(t, J=6.3 Hz, 4H), 3.00 (t, J = 10.1 Hz, 5H), 2.16 - 1.98 (m, 4H). ¹³C NMR (75 MHz, D₂0): δ (ppm) = 155.06, 149.23, 149.10, 122.76, 120.06, 70.59, 69.08, 47.86, 24.31.

ESI(-)-MS: m/z calculated for [C₁₈H₂₂N₂O₈S₂]²⁻ ~458.50/2; found 229.89.

Subsequently, a two-neck round bottom flask equipped with a condenser was charged with a one-to-one MeOH/H₂0 mixture (10 mL) and sodium 3,3'-(([2,2'-bipyridine]-4,4'-diylbis(methylene))bis(oxy))bis(propane-1-sulfonate) (504 mg, 1 mmol, 2 equiv.). The mixture was vigorously stirred at room temperature until complete dissolution of the sulfonated ligand. Then, NaCl (200 mg, 3.4 mmol, 6.8 equiv.) and RuCl₃ • xH₂0 (131 mg, 0.5 mmol, 1 equiv.) were added to the mixture. The reaction mixture was refluxed overnight. During this time, the initially dark green reaction mixture turned brown and then purple. Then, the reaction mixture was filtered, and the filtrate was accurately dried under reduced pressure. The dry crude mixture was treated with diethyl ether (100 mL) and vigorously stirred, until the whole crude mass was reduced to a microcrystalline dark purple powder (no fluorescence under UV light irradiation). The mixture was decanted, and the precipitate was dried under reduced pressure. The crude ruthenium complex (416 mg, 70% yield) was used as such in the next step. The ¹H NMR analysis revealed a very crowded spectrum, due to the presence of both cis and trans ruthenium complexes. The identity of the complex was confirmed via mass analysis.

ESI(-)-HRMS: m/z calculated for C₃₆H₄₄N₄Na₃O₁₆RuS₄ 1156.97; found 1157.70.

Synthesis of compound having formula (II), inner salt (**5**)

A two-neck round bottom flask equipped with a condenser was charged with MeOH (15 mL) and (3) (303 mg, 0.62 mmol, 1.2 equiv.). The mixture was stirred until complete dissolution of the ancillary ligand, then a solution of (**4**) (609 mg, 0.52 mmol) in water (15 mL) was added. The reaction mixture was refluxed overnight. During this time, the initially dark purple non-fluorescent reaction mixture turned dark caramel (orange fluorescence under UV light irradiation). Then, the reaction mixture was dried under reduced pressure. Water (15 mL) was added to the crude mixture. Upon complete dissolution, the water mixture was extracted with CH,Cl; (3x10 mL), and the aqueous phase was accurately evaporated under reduced pressure. The obtained dry crude was treated with diethyl ether (100 mL) and vigorously stirred, until the whole crude mass was reduced to a microcrystalline dark orange powder (red fluorescence under UV light irradiation). The mixture was decanted, and the precipitate was dried under reduced pressure. The crude ruthenium complex (bis(sodium 3,3'-(([2,2'-bipyridine]-4,4'- diylbis(methylene))bis(oxy)) bis(propane-1-sulfonate) (tert-butyl (2-((2-(4-(4'-methyl-[2,2'-bipyridin]-4-yl)butanamido)ethyl)disulfanyl)ethyl) carbamate)ruthenium(II), inner salt) (228 mg, 70% yield) was used as such in the next step.

¹H NMR (300 MHz, CD₃OD): δ (ppm) = 8.67 (s, 4H), 8.60 - 8.55 (m, 2H), 7.80 - 7.70 (m, 4H), 7.70 - 7.60 (m, 2H), 7.50 - 7.45 (m, 4H), 7.40 - 7.30 (m, 2H), 4.75 (s, 8H), 3.79 - 3.70 (m, 8H), 3.00 - 2.70 (m, 16H), 2.59 (s, 3H), 2.30--2.00 (m, 16H), 1.42 (s, 9H).

ESI(-)-HRMS: m/z calculated for C₆₀H₇₈N₈NaO₁₉RuS₆ 1531.26, found 1531.82; calculated for C₆₀H₇₈N₈NaO₁₉RuS₆²⁻ 1508.28/2, found 753.69.

A round bottom flask was charged with a one-to-one MeOH/H₂0 mixture (10 mL), (5) (228 mg, 0.15 mmol), and TFA (5 mL, excess). The mixture was vigorously stirred at room temperature overnight. Then, the reaction mixture was concentrated under reduced pressure. Water (15 mL) was added to the crude mixture. Upon complete dissolution, the water mixture was extracted with CH,Cl; (3x10 mL), and the aqueous phase was accurately evaporated under reduced pressure. The obtained dry crude was treated with diethyl ether (50 mL) and vigorously stirred, until the whole crude mass was reduced to a microcrystalline dark orange powder (red fluorescence under UV light irradiation). The mixture was decanted, and the precipitate was dried under reduced pressure. The obtained deprotected ruthenium complex (6) (209 mg, 91% yield) was used as such in the next step.

¹H NMR (300 MHz, CD₃OD): δ(ppm) = 8.67 (s, 4H), 8.58 (s, 2H), 7.80 - 7.75 (m, 4H), 7.70 = 7.60 (m, 2H), 7.55 -7.45 (m, 4H), 7.40-7.30 (m, 2H), 4.80 - 4.75 (m, 8H), 3.79 - 3.70 (m, 8H), 3.00 - 2.75 (m, 16H), 2.59 (s, 3H), 2.20-2.00 (m, 16H).

ESI(-)-HRMS: m/z calculated for C₅₅H₇₁N₈O₁₇RuS₆ 1409.23, found 1409.52; calculated for C₅₅H₇₁N₈O₁₇RuS₆²⁻ 1408.22/2, found 705.01.

### Synthesis of compound including Ir-complexes having formula (VI)

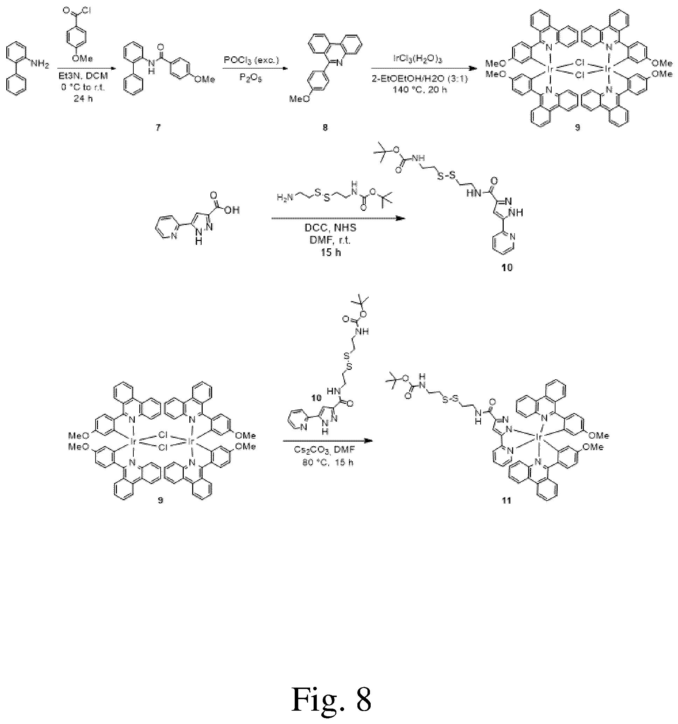

With reference to Figure 8, the reaction pathway for obtaining the compound of formula (VI) is shown.

### Synthesis of (7)

A 250-ml round bottom flask was charged with (commercially available) biphenylamine (5.00 g, 29.55 mmol), 4.12 mL of Et₃N (d = 0.726 g/mL; 2.99 g, 29.55 mmol, 1 equiv) and DCM (40 mL). The resulting mixture was stirred at r.t. until complete dissolution of the biphenylamine starting material and then cooled to 0-5 °C. 4.2 mL of (commercially available) p-methoxy benzoyl chloride (d = 1.36 g/mL; 5.29 g, 31.02 mmol, 1.05 equiv) were added dropwise during 2 min. A precipitate was immediately formed. The reaction mixture thus obtained was allowed to warm up to r.t. and stirred overnight.

Water (30 mL) was added and, after separation of the two layers, the aqueous phase was extracted with DCM (3x30mL). The combined organic phases were dried over Na₂SO₄, filtered and the solvent was removed under reduced pressure. The residue thus obtained was washed with n-hexane and Et₂O yielding a (7) as white solid (6.49 g, 21.4 mmol; y = 72%).

¹H NMR (300 MHz, CDCl₃): δ (ppm) = 8.53 (dd, J = 8.3, 1.2 Hz, 1H), 7.92 (s, 1H), 7.57 - 7.39 (m, 8H), 7.31 - 7.17 (m, 2H), 6.89 - 6.84 (m, 2H), 3.83 (s, 3H).

### Synthesis of (8)

A 100-mL J-Young tube was charged with (7) (3.32g, 10.94 mmol), P₂O₅ (1.55 g, 10.94 mmol, 1 equiv) and 7.2 mL of POCl₃ (d = 1.64 g/mL; 11.75 g, 76.61 mmol, 7 equiv).

The J-Young tap was closed and the reaction mixture was heated to 100 °C and stirred overnight (20 h). After cooling to r.t., excess POCl₃; was removed under reduced pressure and the reaction mixture was taken up with DCM (35 mL). Water (25 mL) and 2M NaOH was added until pH = 10-12 and, after separation of the two layers, the aqueous phase was extracted with DCM (3x30mL). The combined organic phases were dried over Na₂SO₄, filtered and the solvent was removed under reduced pressure.

The residue was washed with *n*-hexane yielding (**8**) as an off-white solid (2.60 g, 10.94 mmol; y = 83%).

¹H NMR (300 MHz, CDCl₃): δ (ppm) = 8.72 - 8.69 (m, 1H), 8.61 (dd, J = 8.1, 1.6 Hz, 1H), 8.26 (d, J = 8.0 Hz, 1H), 8.18 (ddd, J = 8.3, 1.4, 0.6 Hz, 1H), 7.87 (ddd, J = 8.3, 7.0, 1.3 Hz, 1H), 7.78 = 7.60 (m, 5H), 7.12 - 7.06 (m, 2H), 3.92 (s, 3H).

### Synthesis of Iridium dinuclear complex (9)

A 100-mL J-Young tube was charged with IrCl₃(H₂O)₃ (309 mg, 0.88 mmol) and (**8**) (500 mg, 1.75 mmol, 2 equiv). The flask was evacuated for 15 min. 15 mL of 2-EtOEtOH/H₂0 (3:1) were degassed by purging N₂ (15 min) and then added to the reaction mixture under N₂ counter-flow.

The J-Young tap was closed and the reaction mixture was heated to 140 °C and stirred overnight (20 h). The solvent was removed under reduced pressure and then taken up with DMF (12 mL). The DMF suspension thus obtained was filtered on a celite pad (washing with DMF). The solvent was removed under reduced pressure and residue thus obtained was washed with MeOH and Et₂O yielding a brown solid which was proved to be (9) (398 mg, 0.25 mmol; y = 56%) by ¹H, ¹³C NMR and ESI-MS analysis.

¹H NMR (400 MHz, DMSO): δ (ppm) = 9.03 (d, J = 8.4 Hz, 4H), 8.98 (d, J = 8.5 Hz, 4H), 8.86 (d, J = 8.2 Hz, 4H), 8.78 (d, J= 7.9 Hz, 4H), 8.13 = 7.99 (m, 8H), 7.92 (t, J = 7.6 Hz, 4H), 7.68 (t, J = 7.3 Hz, 4H), 7.65 - 7.54 (m, 4H), 6.61 (dd, J = 8.7, 2.4 Hz, 4H), 5.89 (d, J = 2.4 Hz, 4H), 3.31 (s, 12H).

¹³C NMR (101 MHz, DMSO): δ (ppm) = 173.1, 159.1, 151.1, 143.7, 141.5, 133.8, 133.3, 132.8, 129.7, 129.2, 129.2,128.5, 127.0, 124.5,123.5, 123.1, 123.0, 120.8, 107.5, 54.8.

ESI(+)-HRMS: m/z calculated for [(phen)₂Ir]⁺ = [C₄₀H₂₈IrN₂0_{2]}⁺ 761.1780; found 761.1777.

### Synthesis of the ancillary ligand (10)

5-(pyridin-2-yl)-1H-pyrazole-3-carboxylic acid (330 mg, 1.74 mmol), N, N'-dicicloesilcarbodiimmide (DCC) (396 mg, 1.92 mmol, 1.10 equiv) and N-hydroxysuccinimide (NHS) (221 mg, 1.92 mmol, 1.10 equiv) were dissolved in CH₃CN/DMF ((4:1); 25 mL). The resulting mixture was stirred at r.t. for 2 min and then N-Boc-cystamine (396 mg, 1.83 mmol, 1.05 equiv) was added dropwise as DMF solution (5 mL). The reaction mixture was therefore stirred overnight (15 h).

The solvent was evaporated under reduced pressure and the residue was taken up with CH₃CN (20 mL) and filtered (by washing with CH₃CN 3x5mL). The CH₃CN was removed and the residue was taken up with EtOAc (30 mL) and washed with water (20 mL). After separation of the two layers, the aqueous phase was extracted with EtOAc (3x20 mL). The combined organic phases was dried over Na₂SO₄ and filtered. The solvent was removed under reduced pressure and the residue was washed with Et₂0 (2x5mL). An off-white solid was obtained and it was proved to be (**10**) (425 mg, 0.93 mmol; y = 54%) by ¹H, ¹³C NMR and ESI-MS analysis.

¹H NMR (400 MHz, MeOD): δ (ppm) = 8.61 (d, J = 4.9 Hz, 1H), 7.87 (m, 2H), 7.35 (dd, J = 8.7, 4.9 Hz, 1H), 7.26 (s, 1H), 3.71 (t, J = 6.9 Hz, 2H), 3.36 (t, J = 6.8 Hz, 2H), 2.96 (t, J = 6.9 Hz, 2H), 2.83 (t, J = 6.8 Hz, 2H), 1.44 (s, 3H), 1.42 (s, 6H).

¹³C NMR (75 MHz, MeOD): δ (ppm) = 150.4(CH; observable in the HSQC spectrum), 138.7(CH), 124.2(CH), 121.6(CH), 104.5(CH), 40.7(CH₂), 39.9(CH₂), 38.9(CH₂), 39.3(CH₂), 28.7(CH₃). Quaternary carbon resonances were not observed.

ESI(-)-MS: m/z calculated for [C₁₈H₂₅N₅O₃S₂] 423.1399; found 422.31 [M-H]⁻. ESI(+)-MS: m/z calculated for [C₁₈H₂₅N₅O₃S₂] 423.1399; found 446.26 [M+Na]⁺.

### Synthesis of compound having formula (IV) (11)

A 100-mL J-Young tube was charged with (**9**) (50 mg, 0.031 mmol), (**10**) (30.6 mg, 0.07 mmol, 2.3 equiv) and Cs₂CO₃ (10 mg, 0.031 mmol, 1.0 equiv). The flask was evacuated for 15 min. 3 mL of DMF were degassed by purging N₂ (15 min) and then added to the reaction mixture under N₂ counter-flow.

The J-Young tap was closed and the reaction mixture was heated to 80 °C and stirred overnight (15 h). After cooling to r.t., the reaction mixture was filtered over a celite pad (washing with DMF). The supernatant was syringe-filtered (20 um) and the DMF removed under reduced pressure. The residue thus obtained was washed with Et₂O giving a red solid which was proved to be (**11**) (72 mg, 0.06 mmol; y = 97%) by ¹H NMR, ESIMS analysis. ¹H NMR (300 MHz, DMSO): δ (ppm) = 9.18 (d, J = 8.3 Hz, 1H), 9.07 (d, J = 7.9 Hz, 1H), 8.79 (d, J = 7.6 Hz, 1H),8.71(d, J=8.2 Hz, 1H), 8.53 (t, J = 8.2 Hz, 2H), 8.36 -8.27 (m, 3H), 8.15 (d, J = 8.9 Hz, 1H), 8.00 - 7.74 (m, 3H), 7.69 (d, J = 8.8 Hz, 1H), 7.62 - 7.48 (m, 2H), 7.45 - 7.28 (m, 4H), 7.16 (d, J = 8.8 Hz, 1H), 7.04 (m, 3H), 6.85 (dd, J=9.0, 2.7 Hz, 1H), 6.74 (dd, J = 8.9, 2.6 Hz, 1H), 6.56 (s, 1H), 6.35 (d, J = 2.7 Hz, 1H), 5.98 (d, J = 2.6 Hz, 1H), 3.47 (s, 3H), 3.41 (s, 3H), 2.84 - 2.69 (m, 8H), 1.40 (s, 6H), 1.37 (s, 3H). ESI(+)-MS: m/z calculated for [(phen)₂Ir(pyrpyraz)] = C₅₈H₅₂IrN₇O₅S] 1183.31; found 1184.04 [M + H]⁺.

## Claims

1. A compound having a general formula (I)
A-B-C (I)
wherein
A is a luminophore unit in the form of a mono-nuclear, di-nuclear or poly-nuclear metal complex,
B is a linker including a cleavable bond,
C is a conjugating group or an antibody for the sandwich assay.

2. Compound according to claim 1, wherein A is a mono-nuclear metal complex having a general formula (II) 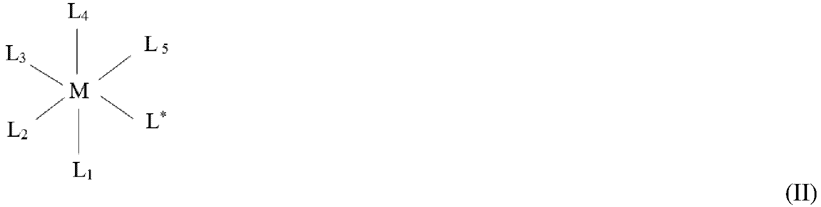 wherein
M is a metal ion
L₁ - L₅ are functional ligands bound to the central metal ion to form the metal complex L* is an ancillary ligand to which linker B is connected.

3. Compound according to claim 1 or 2, wherein A is selected among ruthenium-based complexes and iridium-based complexes.

4. Compound according to claim 1 or 2 or 3, wherein B is a chain including a redox-sensitive bond, cleavable as the result either of oxidation or of reduction.

5. Compound according to claim 4, wherein B includes a bond sensitive to reduction, preferably a disulfide bond.

6. Compound according to any of claims 1 - 5, wherein C is selected from amine (-NH₂), amide, azide (-N3), carboxylate (-COOH), hydroxy (-OH), thiol (-SH), alkyne (-C≡CH), alkene (-CH=CH2) , carbamate (-NH-CO-OR/RNH-CO-O-) , isocyanate (-NCR), tri-alkoxysilane group (-Si(OR)3) carboxyl group, streptavidin or N-hydroxysuccinimide group, aldehyde, carboxylic acid, carboxylic acid ester, epoxide, N- hydroxysuccinimide ester, halogen, hydrazine, hydroxyl, sulfhydryl, maleimido, alkynyl, isothiocyanate or phosphoramidite.

7. Compound according to claim 1, wherein said compound is selected among the compounds according to formulas IV to VI shown below

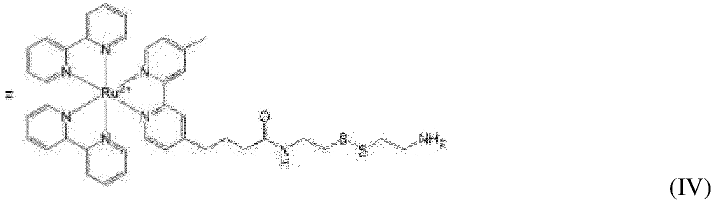

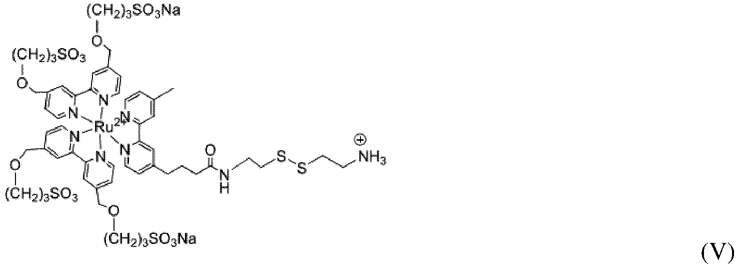

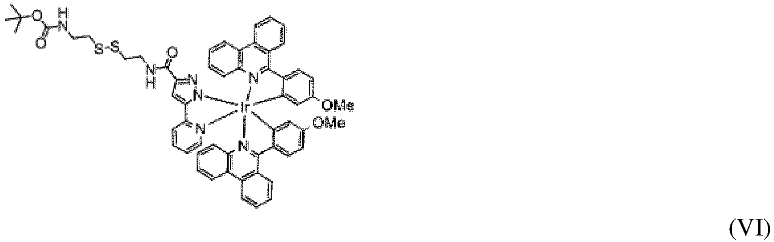

8. Use of the compound of any of claims 1 - 7 for performing an electrochemiluminescence reaction.

9. Use according to claim 8, wherein the electrochemiluminescence reaction is performed in aqueous solution.

10. Use according to claim 8 or 9, wherein a co-reactant is provided.

11. Use according to claim 11, wherein the co-reactant is selected among primary or secondary or tertiary amines, oxalate and hydrogen peroxide.

12. Use according to claim 11, wherein the co-reactant is tri-n-propylamine (TPrA).

13. Use according to claim 12, wherein the concentration of TPrA is 40 - 50 mM.

14. Use of the compound of any of claims 1 - 8 in an electrochemiluminescence-based detection method.

15. Use according to claim 14 for detecting pathogens, bacteria, viruses, parasites, RNA, DNA, biomolecules and other small analytes in biological samples.
